# FASCICULE DE BREVET EUROPEEN

(11) **EP 0 909 815 B1**
(45) Date de publication et mention de la délivrance du brevet: **21.05.2008**
(21) Numéro de dépôt: 98120228.6
(22) Date de dépôt: 20.09.1991
(51) Int. Cl.: C12N 15/05, A01H 1/02, A01H 5/00, C12Q 1/68

(54) **Séquence d'ADN conférant une stérélite mâle cytoplasmique, génome mitochondrial, génome nucléaire, mitochondrie et plante contenant cette séquence, et procédé de préparation d'hybrides**
Cytoplasmatische männliche Sterilität verleihende Dna-Sequenz, mitochondriales Genom, nukleäres Genom, Mitochondrien und diese Sequenz enthaltende Pflanze, und Verfahren zur Herstellung von Hybriden
Dna sequence conferring cytoplasmic male sterility, mitochondrial genome, nuclear genome, mitochondria and plant containing said sequence and process for the preparation of hybrids

(30) Priorité: 21.09.1990 FR 9011670
(43) Date de publication de la demande: 21.04.1999
(62) Demande divisionnaire de: 91919210.4
(73) Titulaire: INSTITUT NATIONAL DE LA RECHERCHE AGRONOMIQUE, 75007 Paris (FR)
(72) Inventeur: Bonhomme, Sandrine, 75015 Paris (FR); Budar, Françoise, 91470 Les Molieres (FR); Lancelin, Dominique, 78530 Buc (FR); Pelletier, Georges, 91440 Bures/Yvette (FR)
(74) Mandataire: Warcoin, Jacques

(56) Documents cités:
- WO-A-87/01726
- WO-A-90/08830
- GB-A- 2 211 205
- PELLETIER, G., ET AL.: "Intergeneric cytoplasmic hybridization in Cruciferae by protoplast fusion" MOLECULAR AND GENERAL GENETICS, vol. 191, 1983, XP002098971
- CHETRIT, P., ET AL.: "Mitochondrial DNA polymorphism induced by protoplast fusion in Cruciferae" THEOR. APPL. GENET., vol. 69, 1985, pages 361-366, XP002098972
- VEDEL, F., ET AL.: "Mitochondrial DNA variation in cytoplasmic male sterile somatic hybrids of Brassica napus" PLANT PHYSIOL. BIOCHEM., vol. 25, no. 3, 1987, pages 249-257, XP002098973
- MAKAROFF, C.A., ET AL.: "The atp6 coding region has been disrupted and a novel reading frame generated in the mitochondrial genome of cytoplasmic male-sterile radish" JOURNAL OF BIOLOGICAL CHEMISTRY, vol. 264, 15 juillet 1989, pages 11706-11713, XP002098974
- BONHOMME, S., ET AL.: "A 2.5kb NcoI fragment of Ogura radish mitochondrial DNA is correlated with cytoplasmic male-sterility in Brassica cybrids" CURR. GENET., vol. 19, no. 2, février 1991, pages 121-127, XP000770810
- CONNETT, M.B., ET AL.: "Plant transformation as a test of the relationship between cytoplasmic male sterility, respirator phenotype, and the PCF gene" J. CELL. BIOCHEM. SUPPL., vol. 13d, 1989, page 299 XP002098975
- JOURDAN ET AL: "Synthesis of male sterile, triazine-resistant Brassica napus by smatic hybridization between cytoplasmic male sterile B.oleracea and atrazine-resistant B.campestris" THEORETICAL AND APPLIED GENETICS, no. 78, 1989, pages 445-455, XP002076791
- MENCZEL ET AL: "Fusion-mediated combination of Ogura-type cytoplasmic male sterility with Brassica napus plastids using X-irradiated CMS protoplasts" PLANT CELL REPORTS, no. 6, 1987, pages 98 -101, XP002076795

## Description

La présente invention se rapporte à un matériel biologique possédant une stérilité mâle utile pour le développement de variétés hybrides d'espèces d'intérêt agronomique.

Elle concerne en particulier une plante appartenant à la famille des cruciféracées dont le cytoplasme des cellules contient des organites possédant des séquences nucléotidiques conférant une stérilité mâle et de bonnes caractéristiques agronomiques.

Le développement de variétés hybrides peut être facilité ou rendu possible par l'utilisation d'un système de stérilité mâle cytoplasmique. Les hybrides sont obtenus par la fécondation croisée entre deux populations parentales, l'une jouant le rôle de mâle, l'autre de femelle. L'un des écueils rencontrés lorsque l'on souhaite obtenir des variétés hybrides de qualité uniforme par croisement sexué sur des espèces autogames, est la capacité de la plante à s'auto-polliniser. Les systèmes de stérilité mâle permettent d'obtenir des plantes femelles incapables de s'autoféconder et sur lesquelles après pollinisation on peut récolter directement les graines qui sont toutes hybrides sans avoir recours à des techniques laborieuses telle que la castration des fleurs.

Parmi les déterminants génétiques de la stérilité mâle, il en est qui sont portés par le cytoplasme. A chaque génération sexuée ils sont transmis exclusivement par la mère. On obtient ainsi 100 % de mâle-stériles à chaque génération avec un système de stérilité-mâle cytoplasmique (CMS). Ces déterminants génétiques sont portés par le génôme des mitochondries.

Un système de stérilité mâle cytoplasmique approprié chez les cruciféracées est défini par les caractéristiques suivantes :
1) La stérilité mâle doit être totale, c'est à dire que quelles que soient les conditions de culture et quelle que soit la lignée que l'on veut utiliser comme parent femelle, il ne doit pas y avoir de production de pollen. Dans le cas contraire, les graines récoltées sur ces plantes femelles seraient en partie issues d'autofécondation et ne seraient donc pas du type hybride F1.
2) La production de ces graines doit être réalisée en profitant des vecteurs naturels du pollen, c'est à dire dans le cas de ces espèces des hyménoptères, des diptères, et le vent. Le pollen doit être transporté des plantes pollinisatrices sur les plantes mâle-stériles (femelles). En fait seuls les insectes peuvent assurer ce transport à distance chez les cruciféracées.
   Les plantes femelles, par conséquent, doivent être suffisamment attractives pour les insectes qui viennent y chercher le nectar. La morphologie des fleurs doit contraindre l'insecte à effectuer cette recherche par le sommet de la fleur de façon à ce que son thorax, principalement, entre en contact avec le stigmate. Pratiquement cela revient à dire que la base des pétales doit former une sorte de tube autour de la base du pistil.
3) La morphologie des organes femelles (pistil) doit être identique à celle d'une plante fertile, en particulier un seul pistil par fleur et de forme rectiligne. En effet, il arrive souvent que des stérilités mâles se traduisent aussi par une féminisation des anthères qui se transforment en pseudopistils et même par la transformation des nectaires en fleurs complètes. Il arrive aussi que le ou les pistils ainsi produits soient déformés. Toutes ces aberrations ne permettent pas une bonne production de graines, et l'on peut parler d'une certaine stérilité femelle.
4) Pour la production de variétés hybrid-ts F1 chez les espèces dont on récolte les graines, comme le colza ou les moutardes, il est indispensable que le parent mâle de l'hybride annule totalement l'effet du cytoplasme mâle stérile, de telle sorte que les plantes hybrides soient facilement pollinisées.

Chez Tes Cruciféracées le premier cas de cytoplasme mâle stérile ou CMS a été décrit par Ogura (1968) chez le radis, Raphanus sativus. Bannerot (1974, 1977) a transféré le cytoplasme Ogura chez les Brassicae, obtenant ainsi des plantes qui possèdent une stérilité mâle cytoplasmique. Ces mêmes plantes ne présentaient pas des caractéristiques agronomiques satisfaisantes (chlorose lors de l'abaissement des températures, mauvaise fertilité femelle) d'où un mauvais rendement les rendant inaptes à l'utilisation commerciale.

Chez les crucifères pour éviter cette chlorose, il convient d'associer dans la même cellule les génomes nucléaires et chloroplastiques d'un même genre. Ainsi des plantes de Brassicae possèdant l'un des génômes chloroplastiques des Brassicae ne présentent plus de chlorose. Si elles possèdent la totalité du génôme mitochondrial Ogura elles présentent une stérilité mâle cytoplasmique totale mais cependant les fleurs auront une morphologie aberrante rendant impossible leur pollinisation par les vecteurs naturels.

En outre, pour les espèces où l'intérêt réside dans les graines, il convient de restaurer la fertilité mâle des variétés hybrides que l'on commercialise par l'intermédiaire de gènes nucléaires dits restaurateurs.

Il est difficile de restaurer la fertilité mâle de plantes possédant la totalité du génôme mitochondrial Ogura car il est nécessaire de faire intervenir simultanément plusieurs gènes restaurateurs.

Nous nous sommes proposé d'obtenir un système de stérilité mâle approprié en éliminant les gènes responsables des caractères indésirables du cytoplasme Ogura tout en gardant une stérilité mâle efficace et facile à restaurer.

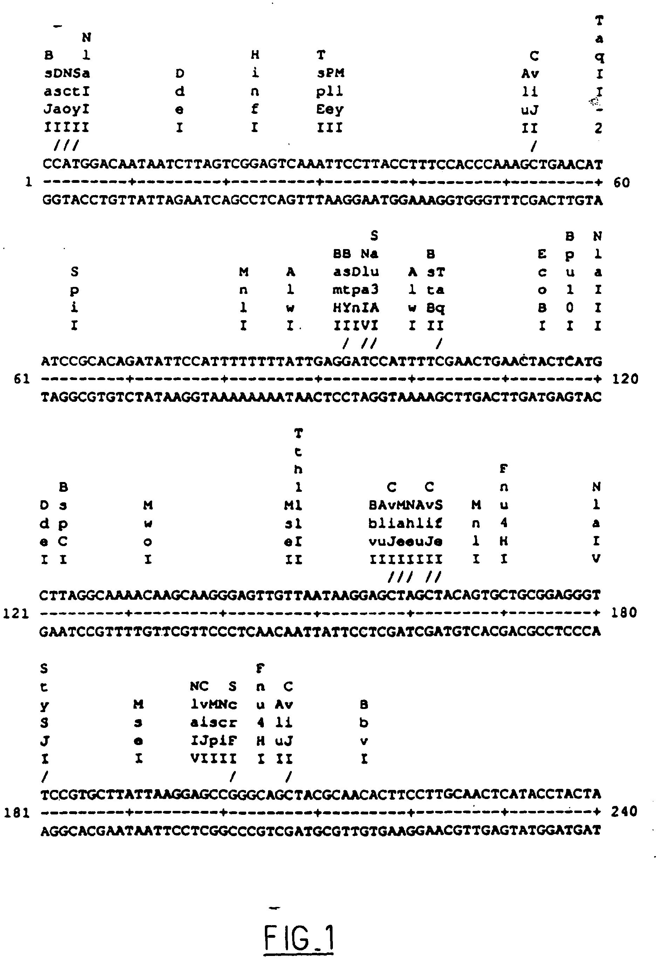

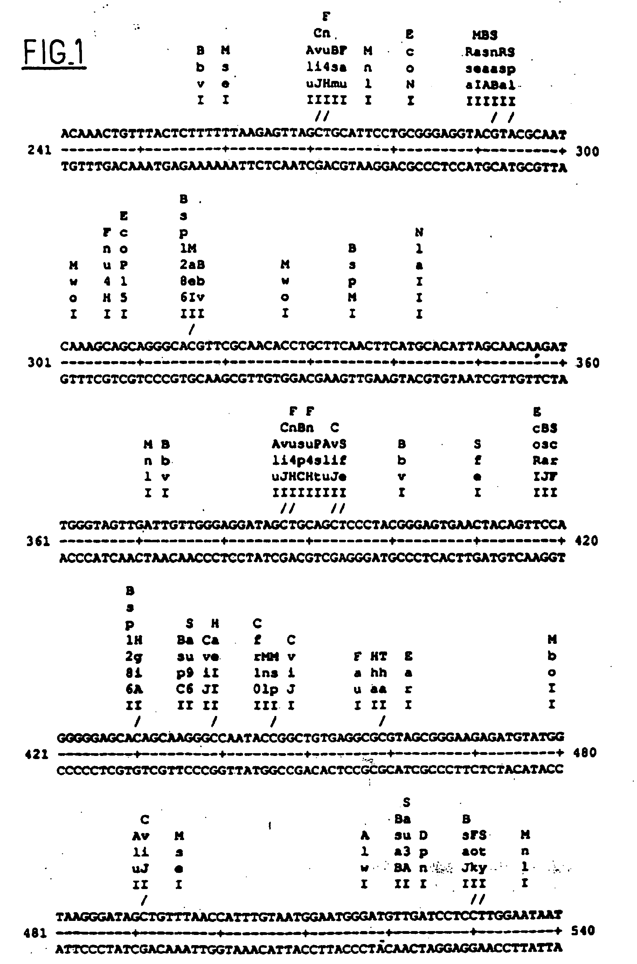

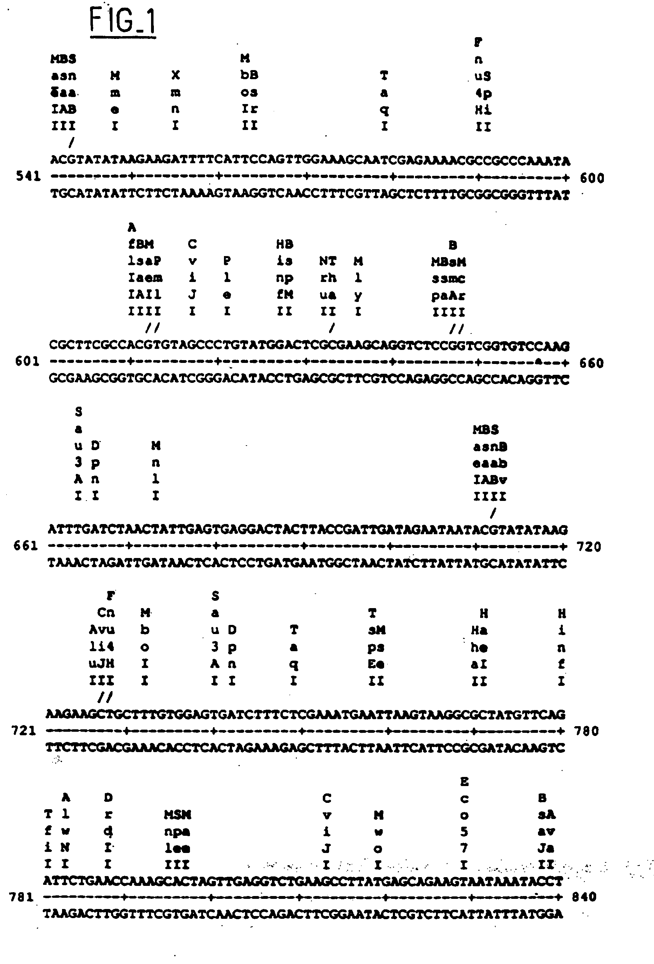

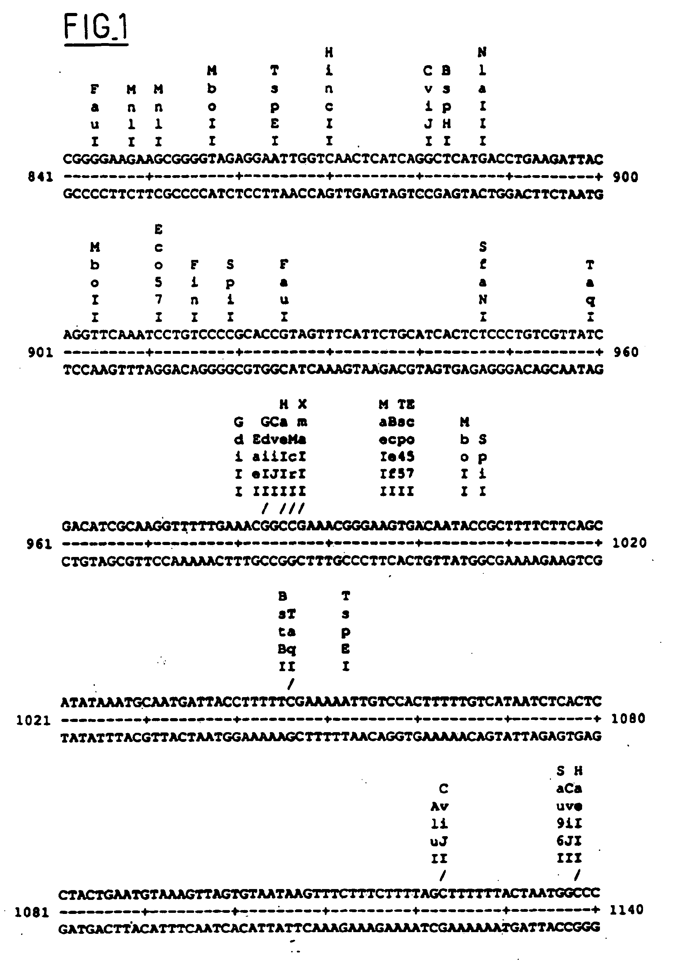

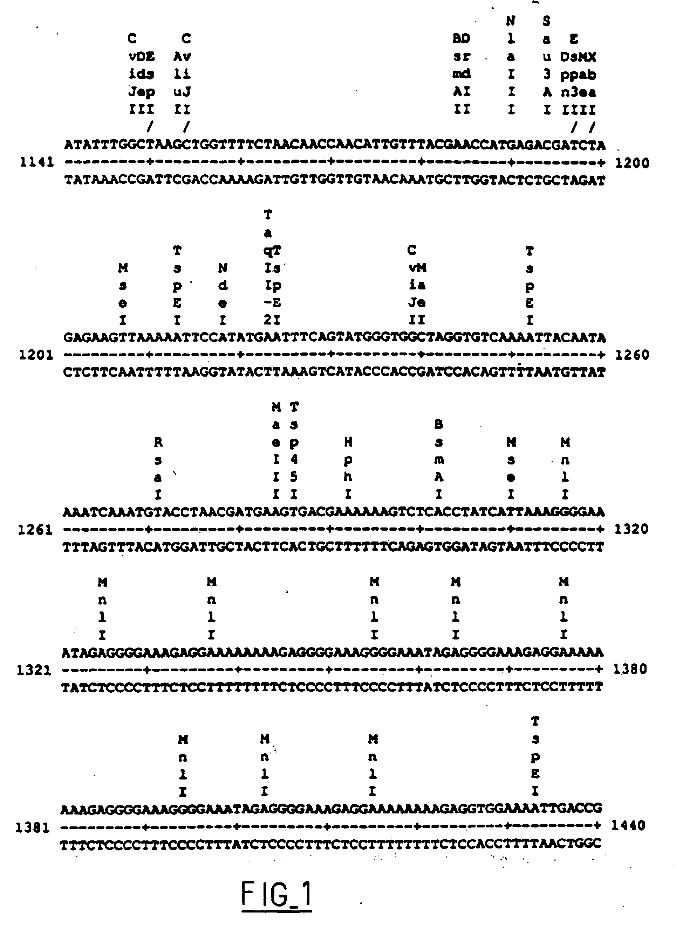

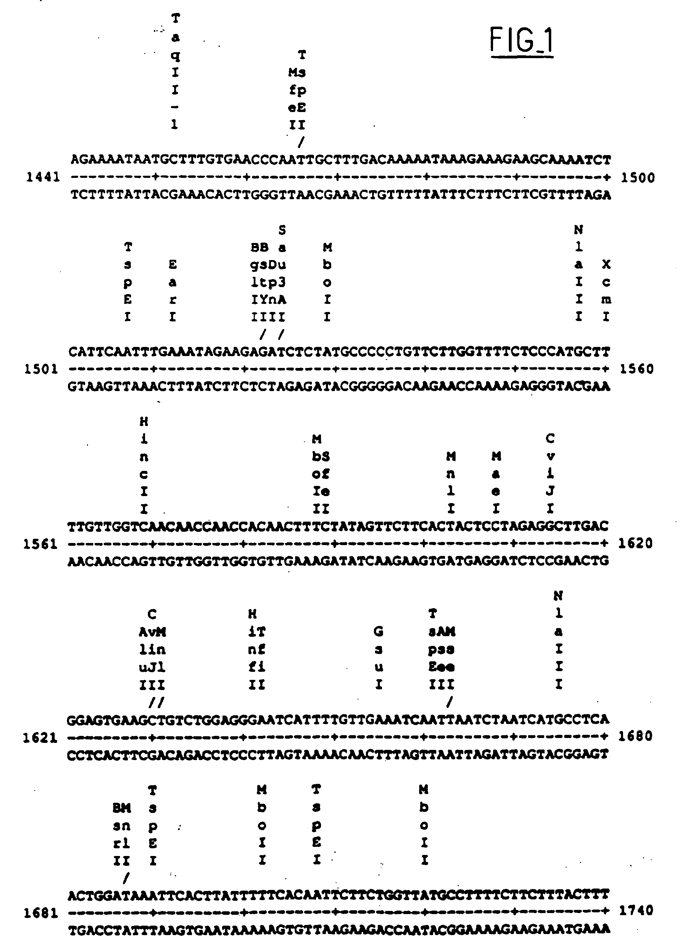

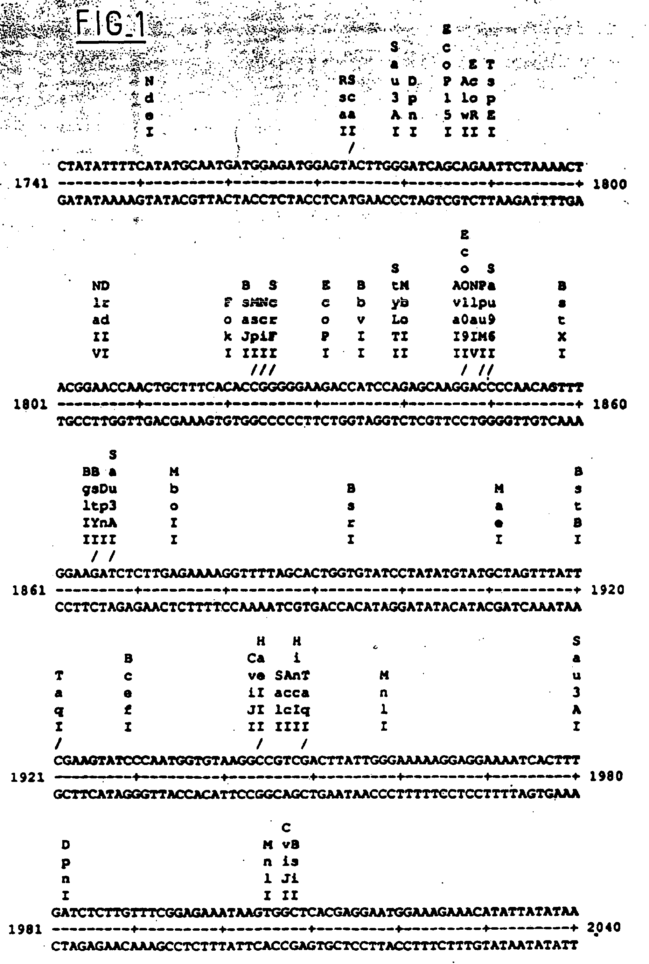

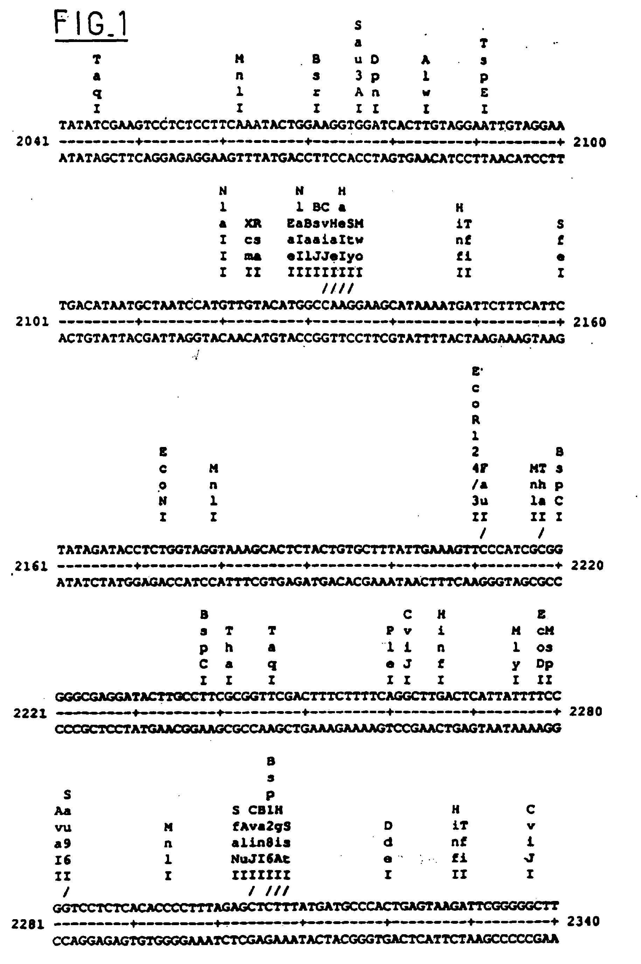

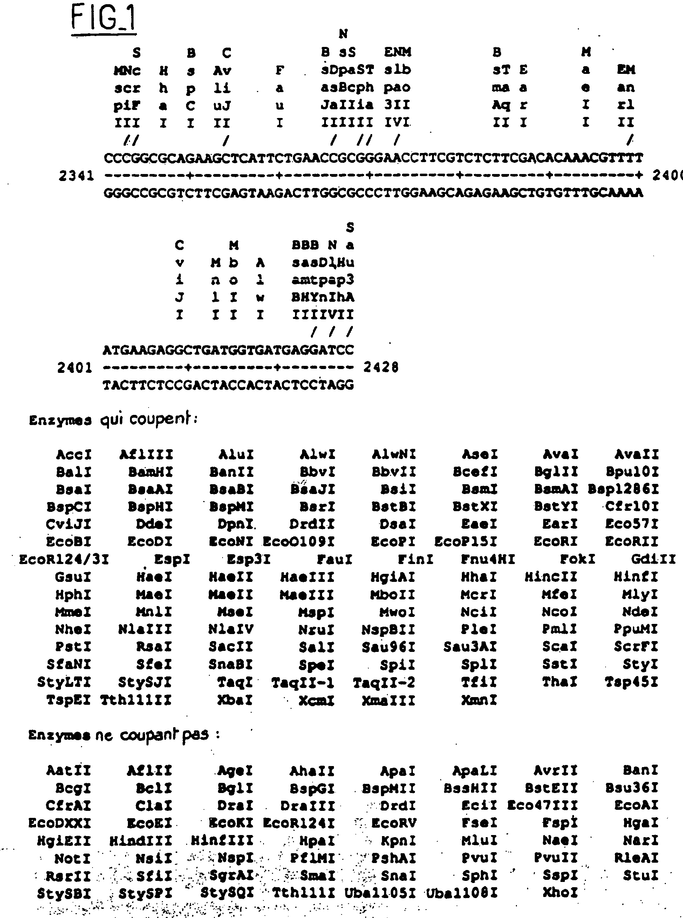

C'est pourquoi la présente invention se rapporte à une séquence d'ADN que nous appellerons "stérilité Ogura", caractérisée en ce que :
a) elle est portée par une séquence d'ADN délimitée par les nucléotides 928 et 2273 de la figure 1, ou
b) elle présente au moins 50 % d'homologie avec ladite séquence mentionnée en a),
et confère, lorsqu'elle est présente dans le génôme mitochondrial ou nucléaire d'une plante, une stérilité mâle cytoplasmique à ladite plante.

En particulier, la présente invention a pour objet une séquence d'ADN stérilité Ogura, caractérisée en ce que :
c) elle est portée par la séquence délimitée par les nucléotides 928 et 1569 de la figure 1, ou
d) elle présente au moins 50 % d'homologie avec ladite séquence mentionnée en c)
et en ce qu'elle est transcrite en ARN dans les mitochondries de plantes mâle-stériles

Dans ce qui suit, on se référera aux figures suivantes :
FIGURE 1 : Séquence nucléotidique du fragment d'ADN mitochondrial de radis Ogura portant le caractère CMS.
FIGURE 2 : Carte de restriction du fragment d'ADN mitochondrial décrit fig. 1.
FIGURE 3 : Electrophorèse d'ADN mitochondrial après digestion par Bg11 (3a) et Nrul (3b). Les bandes révélées correspondent à une hybridation avec une sonde Coxl. (Hiesel et al. 1987).
FIGURE 4 : Electrophorèse d'ADN mitochondrial après digestion par Sall. Les bandes révélées correspondent à une hybridation avec une sonde délimitée par les nucléotides n° 389 et 1199 de la séquence décrite fig. 1.
FIGURE 5 : Fruits produits par des plantes de chou portant différents génômes cytoplasmiques.
FIGURE 6 : Electrophorèse d'ARN mitochondrial. Les bandes révélées correspondent à une hybridation avec une sonde, fragment EcoRI - Bam HI, incluant une partie de la séquence dite ORF B.

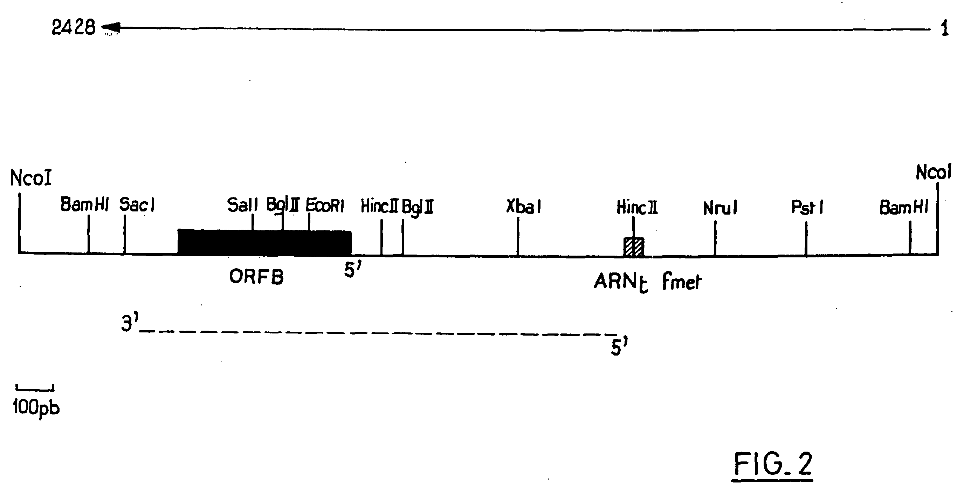

La séquence d'ADN stérilité Ogura est définie par rapport à la séquence délimitée par les nombres 1 et 2428 sur la figure 1. Elle est portée par une séquence transcrite dont les extrémités 3' et 5' sont reliées par un pointillé sur la figure 2, et qui est observée uniquement chez les plantes mâle-stériles. ORF B correspond à un cadre de lecture ouvert ; cette appellation a été donnée d'après l'homologie observée avec une séquence décrite par Brennicke. Sur la figure 2, on a représenté en hachuré la séquence correspondant à l'un des deux gènes de l'ARN de transfert formylméthionine.

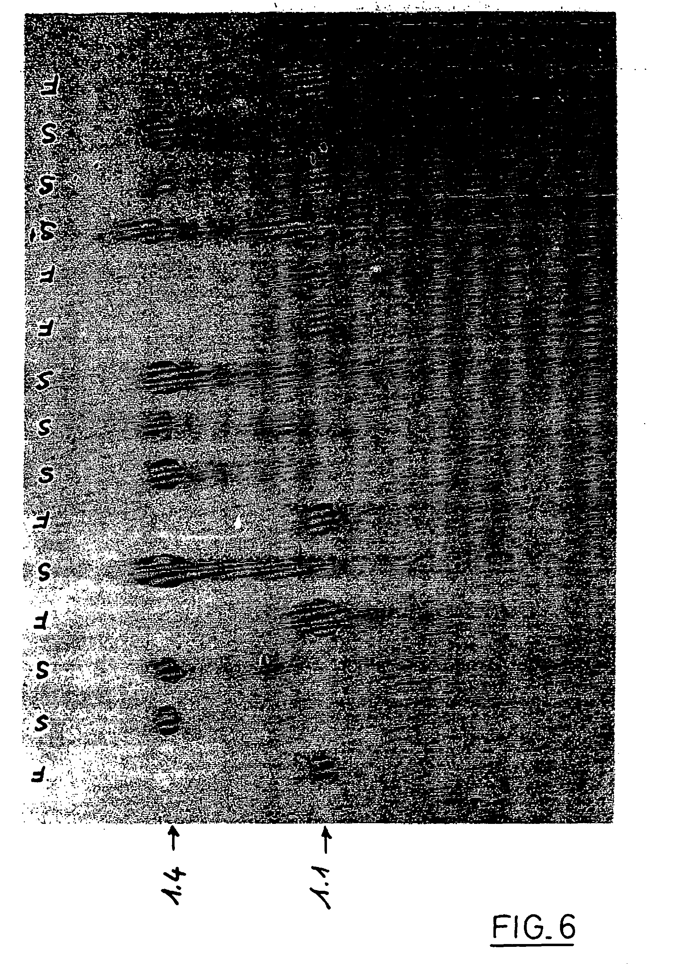

La séquence d'ADN délimitée par les nucléotides numérotés 928 et 2273 sur la figure 1 correspond à un transcrit qui peut être visualisé par hybridation moléculaire (1,4) comme on le voit sur la figure 6. Sur cette figure 6, chaque puits correspond à une plante fertile (F) ou mâle-stérile (S). Seules les plantes mâle-stériles synthétisent un transcrit d'environ 1400 bases. Ce transcrit débute à la position 928 (± 10 bases) de la séquence figure 1 et se termine à la position 2273 (± 5) (l'initiation et l'arrêt de transcription peuvent se produire à différentes positions dans les mitochondries végétales).

De manière préférée, la présente invention se rapporte à un cytoplasme comportant une séquence d'ADN présentant au moins 50 % d'homologie avec la séquence délimitée par les nucléotides 928 et 2273 de la figure 1 conférant le caractère CMS ou un cytoplasme comportant une séquence d'ADN présentant au moins 50 % d'homologie avec la séquence délimitée par les nucléotides 928 et 1569 de la figure 1, et transcrite en ARN, conférant le caractère CMS et caractérisé en ce qu'il comporte :
- des chloroplastes de la même espèce que le génôme nucléaire ou d'une autre espèce mais compatibles avec ce génôme nucléaire,
- ne comporte pas tout ou partie de l'un ou l'autre (ou des deux) fragments du génôme mitochondrial Ogura, défini ci-après :
   - portant l'un des deux gènes de l'ARN de transfert formylméthionine servant à l'initiation de la traduction,
   - portant le gène Coxl, codant pour la sous unité n° 1 de la cytochrome oxydase.

L'absence de ces fragments, dits "séquences indésirables", est nécessaire pour obtenir des génômes mitochondriaux correspondant à une stérilité mâle de bonne qualité, répondant aux 4 caractéristiques définies plus haut.

Selon un autre de ses aspects, l'invention se rapporte à un génôme nucléaire ou mitochondrial végétal recombiné, caractérisé en ce qu'il comporte une séquence d'ADN stérilité Ogura,
a) qui est portée par une séquence d'ADN comprise entre les nucléotides 928 et 2273 de la séquence représentée sur la figure 1, ou
b) qui présente au moins 50 % d'homologie avec ladite séquence mentionnée en a),
et confère lorsqu'il est présent dans le cytoplasme d'une plante, une stérilité mâle cytoplasmique à ladite plante.

En particulier, l'un des objets de la présente invention, est un génome nucléaire ou mitochondrial végétal recombiné, caractérisé en ce qu'il comporte une séquence d'ADN stérilité Ogura,
c) qui est portée par une séquence délimitée par les nucléotides numérotés 928 et 1569 sur la figure 1,
d) qui présente au moins 50 % d'homologie avec ladite séquence mentionnée en c)
et confère, lorsqu'elle est présente dans le cytoplasme d'une plante et est transcrite en ARN, une stérilité mâle cytoplasmique à ladite plante.

Un génôme nucléaire ou mitochondrial selon l'invention peut être caractérisé en ce que ledit génôme recombiné est dépourvu de tout ou partie des fragments de génôme Ogura :
- portant l'un des deux gènes de l'ARN de transfert formylméthionine servant à l'initiation de la traduction,
- portant le gène coxl, codant pour la sous unité n°1 de la cytochrome oxydase,
ou dans lequel lesdits fragments sont inactifs.

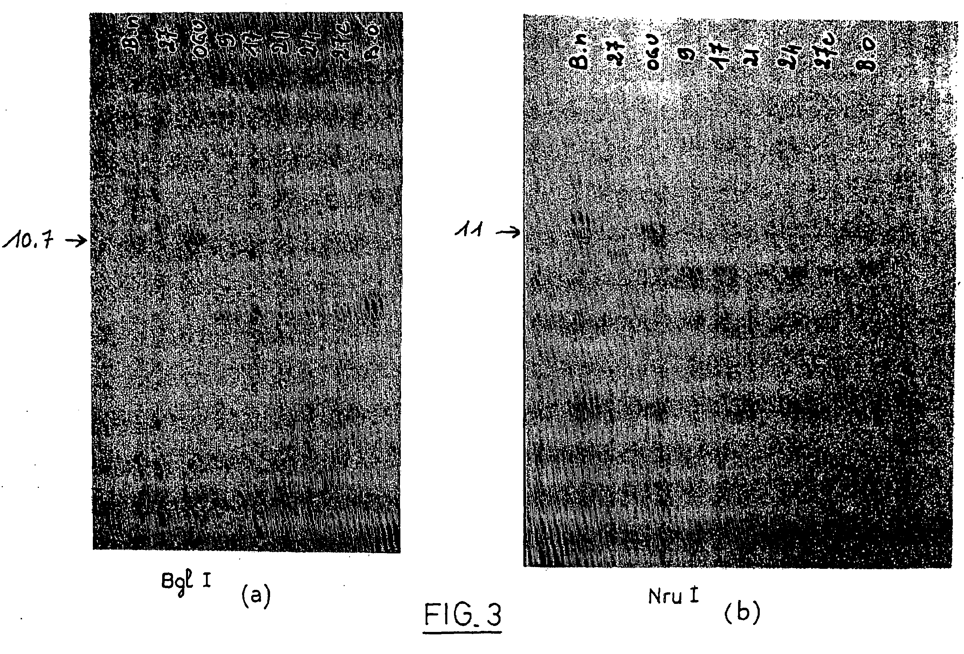

Plus précisément, un génôme nucléaire ou mitochondrial recombiné selon l'invention peut être caractérisé:
1) en ce qu'il est dépourvu de tout ou partie d'un fragment d'environ 10,7 kb après digestion par Bgll ou d'un fragment d'environ Il kb après digestion par Nrul, porteurs du gène coxl.
   Ceci est mis en évidence notamment sur la figure 3, par hybridation moléculaire avec une sonde portant la séquence Coxl.
2) en ce qu'il est dépourvu de tout ou partie d'un fragment de 5,1 kb après digestion par Sall, ou d'un fragment d'environ 15 kb après digestion par Nrul, ou environ 18,5 kb après digestion par Bgll, porteurs de l'un des deux gènes de l'ARN de transfert formylméthionine.

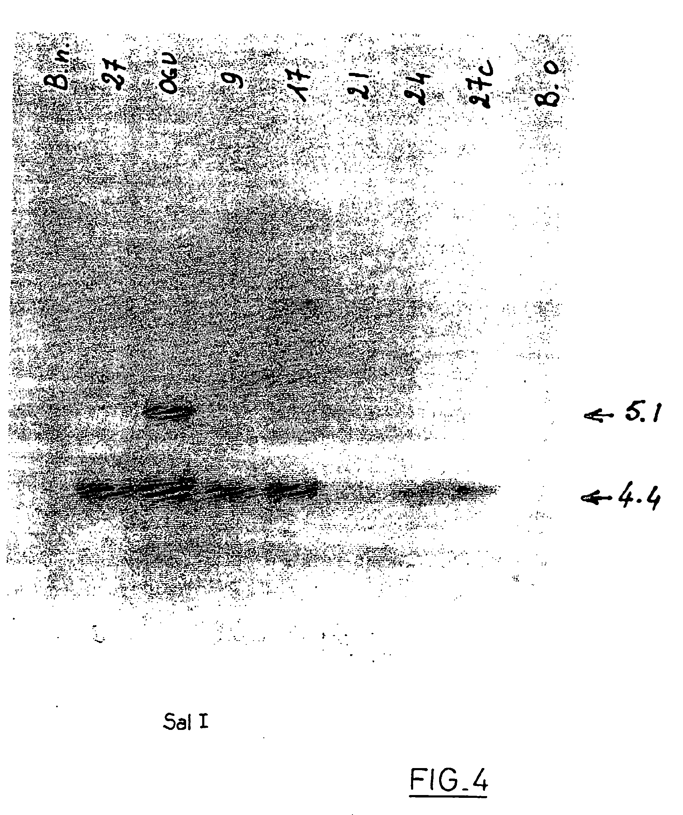

Ceci est mis en évidence notamment sur la figure 4, par hybridation moléculaire avec une sonde délimitée par les nucléotides n° 389 et 1199 de la séquence décrite figure 1.

Sur les figures 3 et 4, les génotypes désignés par des chiffres correspondent à des plantes présentant un système de stérilité mâle cytoplasmique approprié.

| GENOTYPES | CHLOROPLASTES | MITOCHONDRIES |
|---|---|---|
| B.n | B. napus | B. napus |
| 27 | B. napus | B. napus/Ogura |
| OGU. | R. sativus (OGU) | R. sativus (OGU) |
| 9, 17, 21, 24, 27c | B. oleracea | B. oleracea/Ogura |
| B.o | B. oleracea | B. oleracea |

Par ailleurs, l'existence d'un caractère CMS de bonne qualité nécessite la présence d'une séquence d'ADN qui peut être repérée par des hybridations ADN/ADN sur des digestions. C'est ainsi que la présente invention se rapporte à une séquence d'ADN telle qu'elle a pu être définie et caractérisée en ce qu'elle comporte une séquence qui après digestion par Ncol donne un fragment de 2,5 kb, après digestion par Nrul donne un fragment de 6,8 kb, et après digestion par Sall donne un fragment de 4,4 kb.

Cette séquence peut également être repérée par des hybridations sur l'ARN total de plantes mâle-stériles. Un transcrit d'environ 1400 paires de bases est mis en évidence. Il est absent des plantes retournant à la fertilité.

La définition des séquences nucléotidiques "indésirables" et de séquences nucléotidiques "indispensables" à une stérilité Ogura selon l'invention permet de sélectionner, par des techniques d'hybridation d'ADN connues de l'homme de métier, un matériel végétal portant des chloroplastes compatibles avec le génôme nucléaire et portant des mitochondries de bonne qualité sans attendre d'avoir une plante adulte et l'apparition des fleurs et des fruits. On a donc un outil très performant pour sélectionner des plantes possédant un cytoplasme mâle-stérile ayant de bonnes caractéristiques agronomiques.

Selon un autre aspect, l'invention se rapporte à une mitochondrie caractérisée en ce qu'elle contient une séquence nucléotidique correspondant à un ADN présentant au moins 50 % d'homologie avec la séquence délimitée par les bases numérotées 928 et 2273 sur la figure 1 et codant pour la stérilité mâle cytoplasmique d'ogura ; ou bien la mitochondrie contient une séquence d'ADN portée par la séquence délimitée par les nucléotides 928 à 1569 de la figure 1 ou présentant 50 % d'homologie avec cette séquence, et qui est transcrite en ARN dans les mitochondries de plantes mâle-stériles. Cet ADN peut présenter en outre les caractéristiques définies plus haut, notamment l'absence des séquences indésirables.

La présente invention se rapporte également à un cytoplasme de Cruciféracée, caractérisé en ce qu'il comporte une séquence d'ADN "stérilité Ogura" présente dans le génôme mitochondrial ; ce cytoplasme contient en outre des chloroplastes de la même espèce ou d'une autre espèce mais compatibles avec le génôme nucléaire.

La séquence stérilité Ogura est caractérisée en ce que :
a) elle est portée par la séquence d'ADN de 2428 paires de bases représentée sur la figure 1,
b) elle est délimitée par les nucléotides 928 et 2273 de la figure 1 et correspond à un transcrit indiqué par les pointillés figure 2 et visualisé par hybridation moléculaire (1,4) figure 6,
c) elle présente au moins 50 % d'homologie avec ladite séquence mentionnée en b), et confère lorsqu'elle est présente dans le génomes mitochondrial d'une plante, une stérilité mâle cytoplasmique à ladite plante,
   ou
d) elle est portée par la séquence délimitée par les nucléotides 928 à 1569 de la figure 1 et est transcrite en ARN dans les mitochondries de plantes stériles, ou
e) elle présente au moins 50 % d'homologie avec la séquence décrite en d) et est transcrite en ARN dans les mitochondries de plantes stériles.

La présente invention se rapporte également à une plante de la famille des Cruciféracées, caractérisée en ce qu'elle contient des chloroplastes et un noyau de la même espèce ou compatibles, et des mitochondries portant un génôme conférant le caractère CMS tel que défini ci-dessus.

Plus précisément, la présente invention se rapporte également à une plante appartenant au genre Brassica, caractérisée en ce qu'elle contient des chloroplastes et un noyau de Brassica, et des mitochondries portant un génôme conférant le caractère CMS tel qu'il a été défini ci-dessus.

Ce génôme mitochondrial doit également porter un certain nombre de gènes de l'espèce Brassica considérée. Ceci est obtenu par recombinaison entre le génôme Ogura et le génôme Brassica.

En particulier, la présente invention se rapporte à une plante appartenant à l'espèce Brassica napus, caractérisée en ce qu'elle comporte un noyau de Brassica, et que le cytoplasme renferme des chloroplastes de Brassica et des mitochondries mâle-stériles portant un ADN selon la présente invention tel qu'il a été défini plus haut ; ces mitochondries peuvent porter également la plupart des gènes mitochondriaux de Brassica napus (185, Atp9, Atp6, CoxII, ndhl, cob). Brassica napus correspond au Colza, ou Canola et Rutabaga.

La présente invention se rapporte à une plante de l'espèce Brassica oleracea, caractérisée en ce qu'elle comporte un noyau de Brassica et en ce que le cytoplasme renferme des chloroplastes de Brassica et des mitochondries comportant une séquence d'ADN codant pour le caractère CMS telle qu'elle a été définie.

Brassica oleracea recouvre les divers types de choux : choux pommés, de Bruxelles, raves, brocolis, fourragers et choux fleurs.

La présente invention se rapporte également à une plante de l'espèce Brassica campestris, caractérisée en ce qu'elle comporte un noyau de Brassica et en ce que le cytoplasme renferme des chloroplastes de Brassica compatibles avec le génôme nucléaire et des mitochondries comportant une séquence d'ADN codant pour le caractère CMS telle qu'elle a été définie.
Brassica campestris correspond à navette, navets, choux chinois, de Pékin et japonais.

De manière analogue, la présente invention se rapporte à une plante choisie dans le groupe comprenant : B. juncea, B. nigra, B. hirta, carinata, caractérisée en ce qu'elle comporte un noyau de Brassica et en ce que le cytoplasme renferme des chloroplastes de Brassica compatibles avec le génôme nucléaire et des mitochondries comportant une séquence d'ADN codant pour le caractère CMS telle qu'elle a été définie.

Selon un autre de ses aspects, la présente invention a pour objet une plante appartenant au genre Brassica et dont le génome nucléaire comporte une séquence stérilité Ogura telle que définie ci-dessus, ainsi que des éléments assurant son expression et le transport du produit de traduction dans la mitochondrie. Cette plante peut notamment appartenir à l'une des espèces suivantes : B. napus, B. oleracea, B. campestris, B. nigra, B. juncea, B. hirta et B. carinata.

La présence de la "séquence stérilité Ogura" est nécessaire et suffisante pour induire une absence totale de pollen en l'absence de gènes de restauration. La pollinisation de ces plantes est assurée normalement grâce à une bonne production de nectar.

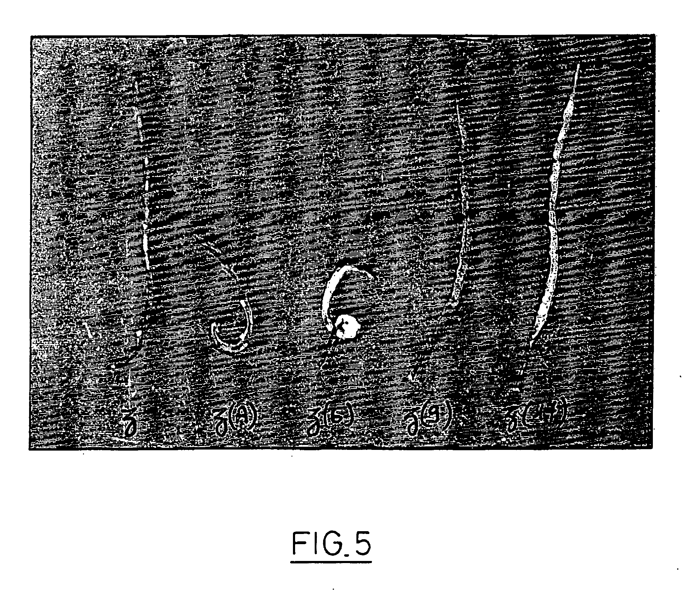

La morphologie des organes femelles est normale et les fruits (siliques) formés contiennent un nombre normal de graines. La figure 5 montre la morphologie observée chez une plante normale témoin (z), une plante à morphologie aberrante possédant le génôme Ogura entier (z(6)) et des chloroplastes Brassica oleracea, une plante de chou portant des chloroplastes Brassica napus et des mitochondries mâle-stériles ayant des gènes Brassica napus (z(A)), et des plantes portant des chloroplastes Brassica oleracea et des mitochondries recombinées ne contenant plus les séquences indésirables (z(9) et z(17)). Les plantes ont les caractéristiques suivantes :

| GENOTYPE | CHLOROPLASTES | MITOCHONDRIES |
|---|---|---|
| z | B. oleracea | B. oleracea |
| z(A) | B. napus | B. napus/Ogura |
| z(6) | B. oleracea | Ogura |
| z(9) | B. oleracea | B. oleracea/Ogura |
| z(17) | B. oleracea | B. oleracea/Ogura |

Les génotypes z(A) et z(6) ne présentent pas un système de stérilité mâle cytoplasmique approprié.

De telles plantes peuvent être obtenues par exemple par la technique de fusion de protoplastes ou par toutes autres techniques qui assurent une bonne recombinaison entre le génôme mitochondrial de l'espèce considérée et le génôme mitochondrial Ogura. Chez de telles plantes la fertilité est restaurée par un seul gène de restauration, appelé Rf1, provenant du radis, ce qui n'est pas le cas des plantes qui portent la totalité du génôme mitochondrial non approprié.

De telles plantes peuvent également être obtenues par reproduction sexuée naturelle ou artificielle.

Des plantes possédant un génôme mitochondrial selon l'invention peuvent également être obtenues par transfert de gène dans la mitochondrie.

Dans tous les cas, ces plantes possèdent un système CMS approprié à savoir :
- une stérilité mâle totale,
- une morphologie permettant une bonne pollinisation et une bonne production de graines comme illustré tableau 1 et tableau 2.

C'est pourquoi la présente invention concerne également un procédé de préparation de plantes hybrides, caractérisé en ce que l'on croise une plante présentant un caractère CMS approprié, comportant la séquence stérilité Ogura dans son génôme mitochondrial ou nucléaire avec une plante normale quand il s'agit d'une culture potagère ou fourragère, ou avec une plante apportant un gène restaurateur de fertilité, Rf1, lorsqu'il s'agit de récolter des graines. Elle concerne également une plante hybride obtenue par ce procédé.

D'une manière générale, les meilleures caractéristiques agronomiques sont obtenues pour des plantes mâle-stériles, possédant des chloroplastes de la même espèce que le noyau et des mitochondries présentant un système de stérilité mâle approprié.

Le tableau 1 représente la productivité d'une lignée de choux sur différents cytoplasmes, (les génotypes z9 ou z 17 sont appropriés). Le tableau 2 représente la productivité d'une lignée de colza sur différents cytoplasmes (les génotypes Fu 27, Fu 58 et Fu 85 sont appropriés).

**TABLEAU 1 : PRODUCTIVITE DE LA LIGNEE z (CHOU A CHOUCROUTE) SUR DIFFERENTS CYTOPLASMES**

| Cytoplasme | | Génotypes | Récolte de semences |
|---|---|---|---|
| Chloroplaste | Mitochondries | | Grammes/plantes |
| B. oleracea | B. oleracea | (z) | 53,1 |
| (témoin fertile) | | | |
| B. napus | Ogura | (zC) | 0 |
| B. napus | Ogura/napus | (zA) | 22,7 |
| B. oleracea | Ogura | (z6) | 9,3 |
| Ogura | Ogura | (z0) | 20, 1 |
| B. oleracea | Ogura/oleracea | (z9 ou z17) | 91,8 |

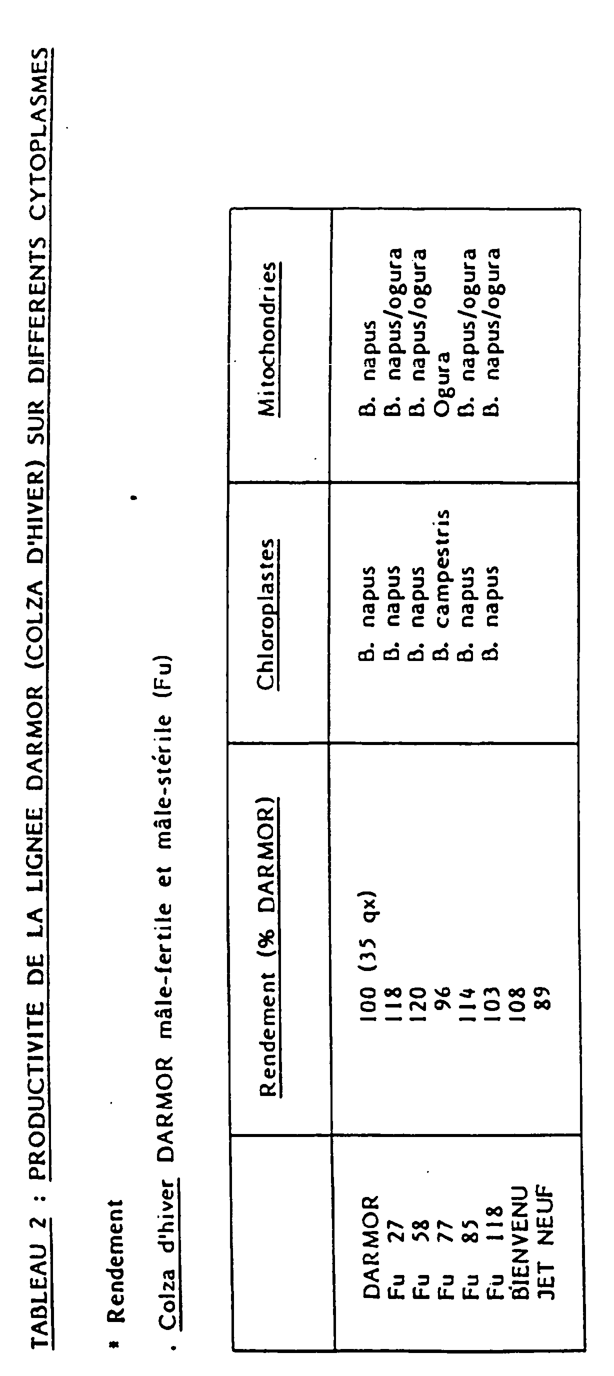

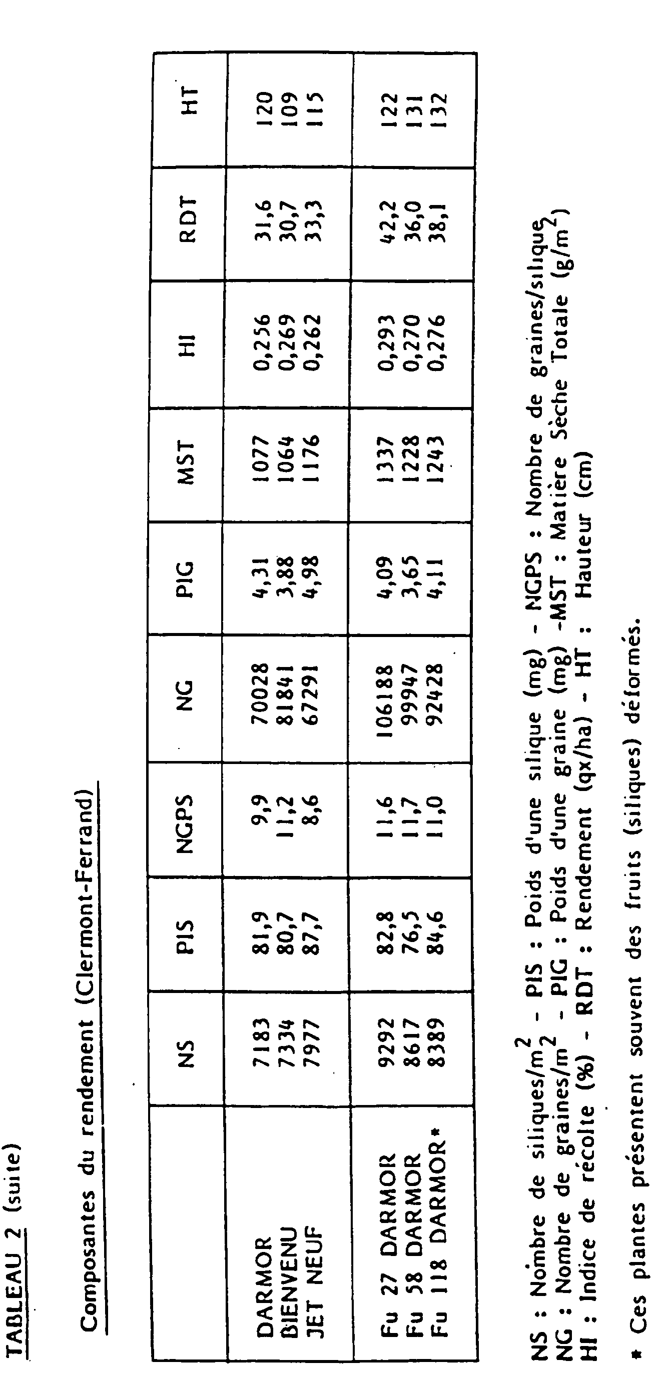

La présente invention a également pour objet une sonde comportant une séquence d'au moins 10 bases, de préférence 15 bases, d'une séquence comprise entre les nucléotides numérotés 928 et 1569 sur la figure 1 ; ladite sonde peut être marquée par exemple au moyen d'une base radioactive ou par tout autre moyen, comme par exemple par fluorescence. Cette sonde peut être utilisée pour la mise en évidence de la stérilité mâle et peut être utilisée notamment dans la sélection de clones.

Des caractéristiques et avantages de la présente invention seront mieux mis en évidence dans les exemples suivants.

### EXEMPLE 1 : MISE EN EVIDENCE DE LA SEQUENCE D'ADN RESPONSABLE DE LA STERILITE MALE CYTOPLASMIQUE OGURA.

### 1. Plante.

Par "cybride" on désigne des formes obtenues par la fusion de protoplastes isolés, suivie par la regénération de la plante entière. Ce mode d'obtention permet le mélange dans la cellule d'informations cytoplasmiques provenant des deux parents. Le cybride n° 13 a été obtenu parmi 820 plantes régénérées par fusions de protoplaste entre un cybride de B. Napus résistant à la triazine, Ogura cms (descendance du cybride 77 décrit dans Pelletier et al., 1983 et Chétrit et al., 1985) et la variété d'origine Brutor, sensible à la triazine, et fertile. Un test de résistance à la triazine (Ducruet et Gasquez, 1978) réalisé sur un échantillon de feuille de chaque régénérant a permis de déterminer le type de chloroplaste (chloroplastes résistants à la triazine provenant du parent 77 ou chloroplastes sensibles à la triazine provenant de la lignée Brutor). On a cultivé les plantes et observé le stade de floraison. Les plantes présentant des combinaisons non parentales (soit sensible/mâle stérile ou résistant/mâle fertile) ont été sélectionnées comme cybrides. Le cybride n° 13 était du type sensible/mâle stérile. Le cybride 1 était du type résistant/mâle fertile.

### 2. Isolement des acides nucléiques.

L'ADN total a été isolé à partir de feuilles provenant de plantes de 4 semaines selon la méthode décrite par Dellaporta (1983). L'ADN mitochondrial a été extrait de feuilles de plantes âgées de 8 semaines ainsi qu'il a été décrit par Vedel et Mathieu, 1982, avec les variantes suivantes :
les mitochondries n'ont pas été purifiées sur gradient de saccharose avant la lyse, et la lyse a été réalisée dans du sarcosyl à 4%, avec protéinase K 0,5 mg/ml (Boehringer Mannheim GmbH), dans du Tris pH 8, 50 mM, EDTA 20 mM. Après précipitation, l'ADN mitochondrial a été purifié par centrifugation en gradient bromure d'éthidium-chlorure de caesium (methode 1- Vedel et Mathieu 1982) dans des tubes de centrifugation en pollyallomère.

Les ARN totaux ont été isolés à partir de feuilles ou de bourgeons floraux selon Logemann et al., 1987.

Les ARN mitochondriaux ont été extraits de choux-fleurs âgés de 8 semaines, selon la technique de Stern et Newton, 1986.

### 3. Analyses par restriction de l'ADN mitochondrial et électrophorèse en gel d'agarose.

Elles ont été réalisées ainsi qu'il est décrit dans Pelletier et al, 1983. Les ARN totaux ou mitochondriaux ont été chargés sur des gels d'électrophorèse contenant du formaldehyde tel qu'il a été décrit par Sambrook et al., 1989.

### 4. Hybridation.

Le transfert d'ADN ou d'ARN sur des filtres de nylon (Hybond-N, Amersham) a été mené par absorption capillaire respectivement avec 6xSSC ou 10xSSPE, suivant les instructions du fabricant. La préhybridation et l'hybridation ont été conduites selon Amersham, en utilisant des sondes marquées par le système de marquage d'ADN multiamorce (Amersham) après purification sur colonnes de Sephadex G50 (Sambrook et al., 1989).

### 5. Clonage de l'ADN mitochondrial.

Deux banques génomiques de lignées cybride mâle stérile (13-7) et révertant (13-6) ont été construites dans un vecteur phage lambda EMBL3, cultivé sur la souche restrictive d'E. Coli Nm539 (Frischauf et al., 1983). On a obtenu environ 2,5 x 10⁴ clones par µg d'ADN mitochondrial.

Les banques d'ADN mitochondrial ont été titrées et étalées afin d'isoler les plaques qui ont été transférées sur les filtres de nylon ainsi qu'il est décrit dans Sambrook et al., 1989. La sonde d'hybridation utilisée pour screener les deux banques d'ADN mitochondrial était préparée comme suit : le fragment d'ADN mitochondrial spécifique de la cms a été élué en utilisant la procédure Gene clean™ (BIO 101 INC.) à partir d'un produit de digestion d'ADN mitochondrial chargé sur un gel d'agarose préparatif. L'ADN élué a été ensuite marqué ainsi qu'il a été décrit.

L'extraction d'ADN Lambda, le sous-clonage du fragment Ncol 2,5 dans le site Ncol de pTrc99A (Amann et al., 1988) et les extractions d'ADN plasmidique ont été menés selon les protocoles de Sambrook et al., 1989. Les plasmides recombinants ont été introduits dans une souche d'E. Coli NM522 (Gough et Murray, 1983).

### 6. Etude génétique du cybride 13 et de ses descendants.

Dans la première génération de descendants obtenus par pollinisation du cybride 13 avec Brutor, composée de 13 plantes, 5 sont totalement mâles stériles (y compris des plantes 13-2 et 13-7), une est mâle fertile (n° 13-6) et 7 sont pratiquement entièrement stériles avec quelques fleurs mâles fertiles.

La plante fertile 13-6 a été auto-pollimsée et croisée avec Brutor. Dans les deux cas on obtient uniquement des plantes fertiles (43 et 42 respectivement).

Dans les croisements entre la plante mâle stérile n° 13-7 et Brutor, 24 descendants sont entièrement stériles et 6 présentent quelques fleurs fertiles, résultat similaire à celui obtenu avec le cybride lui-même. La plante 13-2 a été croisée avec la lignée restauratrice RF qui est hétérozygote pour les gènes spécifiques de restauration pour la stérilité mâle Ogura (Chétrit et al., 1985). La descendance de ce croisement est composée de 53 plantes mâles stériles, 37 plantes mâles fertiles et 9 plantes pratiquement entièrement stériles bien que présentant quelques fleurs fertiles. Ces résultats suggèrent que les plantes mâles stériles de la famille cybride 13 contiennent le déterminant Ogura cms, comme les autres cybrides étudiés auparavant avec un profil de restauration plus simple (Chétrit et al, 1985).

A ce stade de l'étude, deux possibilités peuvent être envisagées: soit le cybride 13 contient un mélange de génomes mitochondriaux mâle fertiles et mâle-stériles, et l'on peut sélectionner davantage pour purifier les deux phénotypes, soit le cybride 13 contient un génome mitochondrial recombiné de structure instable qui retourne à une configuration "fertile" plus stable, et il sera impossible de maintenir un phénotype mâle stérile homogène parmi les générations ultérieures.

Des plantes mâles stériles obtenues à partir de la descendance de la plante mâle stérile n° 13-7 ont été développées à la fois par bouture et par croisement sexué avec Brutor. Après un nombre variable de génération (1 à 5) par les deux méthodes, toutes les familles donnent des plantes fertiles. Par contre, les plantes entièrement fertiles ainsi obtenues ne redonnent jamais de plantes stériles.

A la lumière de ces résultats, on peut considérer que la seconde explication proposée ci-dessus c'est à dire que le cybride 13 porte un génome mitochondrial instable qui perd le déterminant Ogura cms pendant le processus conduisant à une configuration "fertile", sans possibilité de retour à un phénotype stérile, est la bonne.

### 7. Comparaison entre les ADN mitochondriaux de mâle-stériles et de révertants fertiles. Isolement d'un fragment spécifique des plantes mâle-stériles.

L'ADN mitochondrial a été extrait des feuilles de descendants 13-7 mâle-stériles et de révertants fertiles (descendants 13-6 ou 13-7) et digéré avec plusieurs enzymes de restriction, afin de comparer leur profil de restriction. Les génomes mitochondriaux des deux types sont très semblables puisqu'aucune différence ne peut être observée entre les profils de restriction des mitochondries mâle-stériles et révertants fertiles en utilisant différents enzymes. Cependant un fragment de restriction de 6,8 kb a été détecté dans le profil de restriction de l'ADN mitochondrial des plantes mâle-stériles digéré avec Nrul et n'a jamais été observé dans les profils des révertants fertiles correspondants.

Le fragment (appelé N6,8) a été élué d'un gel d'agarose, marqué, et utilisé comme sonde sur des profils de restriction d'ADN mitochondrial Nrul : un signal important à 6,8 kb a été observé chez tous les descendants mâle-stériles du cybride 13, alors qu'aucun fragment de cette taille n'a hybridé à la sonde dans les génomes de mitochondries de révertants fertiles. En outre, la sonde N6,8 hybride avec un fragment de 6,8 kb dans l'ADN mitochondrial Ogura digéré avec Nrul, mais pas dans celui de B. Napus cv Brutor, montrant l'origine Ogura de ce fragment.

Une banque lambda contenant des extraits d'ADN mitochondrial provenant de plantes mâles stériles (13-7) a été testée avec le fragment marqué élué, et parmi 8 clones hybridant, 2 phages recombinants ont été isolés, contenant le fragment N6,8 entier et des séquences adjacentes. Une carte de restriction détaillée de cette région a été obtenue. L'hybridation des profils de restriction de l'ADN mitochondrial provenant de descendants fertiles et stériles du cybride 13 avec N6,8 comme sonde a permis de limiter la région spécifique du génotype mâle stérile à un fragment Ncol de 2,5 kb.

Le fragment Ncol de 2,5 kb a été marqué et utilisé comme sonde vis-à-vis d'ADN mitochondrial provenant de descendants 13-7 et 13-6 digéré avec Ncol. Outre le signal à 2,5 kb qui est spécifique du profil mâle stérile, plusieurs fragments Ncol hybrident à la fois dans les profils de révertant fertile et de mâle-stérile, ces fragments sont à 2,2, 19 et 14 kb. Un fragment Ncol de 2,7 kb hybride fortement dans le génome mitochondrial des descendants fertiles et pas dans celui des descendants stériles. L'analyse de ce profil d'hybridation conduit à la conclusion que le fragment Ncol de 2,5 kb, bien que spécifique de l'ADN mitochondrial mâle-stérile, contient des séquences qui sont répétées ailleurs dans le génome mitochondrial (sur des fragments de 2,2, 10, et 14 kb après digestion par Ncol) et ces séquences répétées sont aussi présentes dans l'ADN mitochondrial de révertants fertiles outre le fragment spécifique de 2,7 kb.

Les ARN totaux sont extraits de feuilles ou de bourgeons de descendants de cybrides 13, ou de cybrides (provenant d'autres expériences de fusion) mâle stérile ou fertile et de lignée Brutor. Les Northern blots ont été réalisés et hybridés avec une sonde correspondant à l'insert du clone Lambda contenant N6,8 décrit dans l'exemple 3. Un transcrit majeur de 1,4 kb a été détecté chez tous les cybrides mâle-stériles, y compris le cybride 13-7, alors qu'on n'a observé aucun transcrit de cette taille dans la lignée Brutor, ni dans les deux cybrides fertiles (différents de 13). Par ailleurs, des plantes fertiles présentent un transcrit de 1,1 kb hybridant avec la sonde qui est absent ou présent à un niveau très bas dans tous les cybrides mâle-stériles testés. Plusieurs transcrits communs à tous les échantillons hybrident faiblement avec la sonde, à cause de la taille importante de l'insert marqué. On a vérifié que les transcrits mitochondriaux peuvent être détectés dans des échantillons d'ARN totaux par hybridation du même Northern blot avec un fragment d'ADN contenant la séquence du gène atpa.

Le même transcrit spécifique de 1,4 a été trouvé dans les ARN mitochondriaux Ogura extraits de choux-fleurs, en utilisant le fragment Ncol2,5 comme sonde. Les limites exactes de ce transcrit ont été déterminées en utilisant comme sonde des sous clones du fragment Ncol 2,5.

### 8. Etude du cybride I et de ses descendants

Le cybride 1 était mâle fertile. Dans sa decendance, la plante 1.12 était fertile et la plante 1.18 stérile. La plante 1.12 a donné dans sa descendance des plantes stériles (S₃) et des plantes fertiles (RF₃). La plante 1.18 a donné des plantes stériles (S₂) et un rameau fertile (RF₂). Les plantes S₂ et S₃ sont restaurées par le même gène nucléaire de restauration de la fertilité pollinique que le cybride 13 stérile.

L'ADN mitochondrial des plantes S2 et 53, par hybridation avec le fragment Ncol de 2,5 kb marqué, ne donne pas un signal à 2,5 kb sur une digestion Ncol, ni un signal à 6,8 kb sur une digestion Nrul.

De même l'hybridation sur les ARN totaux (Northern) avec une sonde correspondant à la séquence ORFB ne donne pas un signal à 1,4 kb cornue chez le cybride 13 stérile. Par contre, une sonde correspondant à la séquence comprise entre les nucléotides 928 et 1569 de la figure 1 donne un signal en Northern à environ 1,3 kb. Ce signal est absent sur l'ARN des plantes RF1, RF2, RF3 ou Brutor. De même il est possible d'utiliser cette séquence (928-1569) comme sonde en dot blot d'ARN totaux et dans ce cas, seules, et toutes, les plantes mâle-stériles donnent un signal.

Ces résultats indiquent que les plantes S2 et S3, bien que mâle-stériles, n'ont pas conservé la séquence nucléotidique, décrite dans la figure 1, dans sa conformation originale et ils démontrent que, dans cette séquence, la partie comprise entre les nucléotides 928 et 1569 est celle qui porte le déterminant spécifique "stérilité Ogura", qui rend les plantes mâle-stériles, lorsque cette séquence est transcrite.

Cette séquence n'a pas d'homologie significative avec les séquences présentes dans les banques de données.

### EXEMPLE 2 : MISE EN EVIDENCE DE SEQUENCES INDESIRABLES DANS LE GENOME MITOCHONDRIAL OGURA.

Une collection de cybrides a été obtenue dans l'espèce B. napus par fusion de protoplastes entre un colza porteur du cytoplasme Ogura et un colza normal. Le premier est mâle-stérile et déficient chlorophyllien à basse température, le second est normalement vert et fertile. Les cybrides ont été triés parmi les plantes régénérées et l'on a retenu ceux qui étaient mâle-stériles et normalement verts.

De la même manière une collection de cybrides a été obtenue dans l'espèce B. oleracea par fusion de protoplastes entre un chou porteur du cytoplasme Ogura et un chou normal. On a retenu parmi les plantes régénérées les cybrides qui étaient mâle-stériles et normalement verts.

Ces cybrides ont été croisés avec différentes variétés, de colza dans le premier cas, de chou dans le second. Les croisements ont été répétés à chaque génération avec les mêmes variétés de façon à obtenir un génotype défini, proche de celui de la variété récurrente.

Ces différentes variétés, converties ainsi sur les cytoplasmes de différents cybrides ont été soumis à des essais agronomiques pour mesurer la production de graines, qui dépend de plusieurs facteurs : une production de nectar suffisante pour assurer une pollinisation par les insectes, une morphologie florale normale pour que cette pollinisation soit efficace et que les fruits se développent normalement.

La collection de cybrides a ainsi pu être divisée en deux lots :
- un lot de cybrides présentant une stérilité mâle appropriée à la production commerciale de semences.
- un lot de cybrides ne présentant pas toutes les caractéristiques favorables à une bonne production commerciale de semences.

Au premier lot appartiennent par exemple les cybrides de colza n° 27, 58, 85 et les cybrides de chou n° 9, 17, 21, 24, 27c.

Au deuxième lot appartiennent par exemples les cybrides de colza n° 23s, 77, 118 et les cybrides de chou n° 1, 6, 14.

Les ADN totaux de ces cybrides ont été soumis à des digestions enzymatique par Sall, Ncol, Nrul, Bgll, Pstl, Kpnl. Les Southern blots obtenus ont été hybridés avec différentes sondes mitochondriales Atpa, Cob, Coxl, Atp6, 26S, 18S, et deux fragments du génôme Ogura de 2,5 kb issu d'une digestion Ncol et de 19,4 kb issu d'une digestion Nrul.

Les deux lots de cybrides se distinguent en ce que :
a) les n° 23s, 77, 11s, chez le colza et 1, 6, 11, chez le chou possèdent la région du génôme Ogura qui entoure le gène coxl reconnaissable par des fragments Bgll de 10.7 kb ou Nrul de 11 kb et la région du génôme Ogura qui entoure un des gènes de l'ARN de transfert formylméthiomine reconnaissable par des fragments Sall de 5,1 kb ou Nrul de 15 kb.
b) les n° 27, 58, 85 chez le colza et 9, 17, 21, 24, 27c chez le chou ne possèdent pas les régions correspondantes qui ont été remplacées, du fait de recombinaisons entre les génômes des deux parents qui ont été fusionnés, par des régions analogues du génôme mitochondrial de colza chez les n° 27, 58, 85, de chou chez les n° 9, 17, 21, 24, 27c.

On en déduit que les deux régions en question du génôme Ogura sont indésirables si l'on veut avoir un système de stérilité mâle approprié à la production commerciale de semences.

### EXEMPLE 3

Cet exemple illustre l'intérêt de la connaissance des séquences "stérilité mâle Ogura" et des séquences indésirables pour effectuer un tri immédiat des cybrides obtenus sans avoir à attendre plusieurs années de rétrocroisement et des tests agronomiques.

On fusionne des protoplastes d'une plante de Brassica portant le cytoplasme Ogura avec des protoplastes de l'espèce Brassica considérée. Les colonies issues de fusion sont cultivées in vitro et mises a régénérer sur un milieu qui favorise la formation de bourgeons (voir Pelletier et al. 1983).

A partir d'un gramme de matière fraîche qu'il s'agisse d'un cal ou d'un fragment de la plantule régénérée, il est possible par les techniques décrites précédemment d'isoler l'ADN total. Après digestion par Sall, l'hybridation de type Southern avec la sonde comprise entre les nucléotides n° 389 et 1199 (voir figure 1) ne doit donner un signal que pour une taille de 4,4 kb (ne doit pas donner de signal à 5,1 kb). De même après digestion par Nrul et hybridation avec une sonde portant le gène coxl, on doit obtenir un signal pour une taille différente de 11 kb.

Ces hybridations permettent de prédire qu'on a bien une plante qui sera mâle stérile et qui sera appropriée à la production commerciale de semence.

### EXEMPLE 4

Cet exemple est une variante de l'exemple 3 ou l'on imagine qu'au lieu de réaliser des fusions de protoplastes, on réalise un croisement sexué entre les deux parents dans des conditions particulières ou avec des génotypes particuliers de telle sorte que, contrairement aux processus connus de la fécondation chez les végétaux, il y a mélange des cytoplasmes de l'oosphère et du tube pollinique ou du gamète mâle. Si de tels procédés étaient décrits, on pourrait de la même manière effectuer un tri précoce, sur de jeunes plantes issues de ces fécondations artificielles en utilisant les mêmes sondes et les mêmes critères que dans l'exemple 3.

### EXEMPLE 5

Cet exemple illustre l'intérêt de la connaissance de la séquence stérilité Ogura dans un type de manipulation génétique qui a déjà été décrit chez la levure (Johnston et al. 1988).

A partir d'une plante de Brassica normale on bombarde des méristèmes ou bien des cellules in vitro avec des microparticules recouvertes d'ADN portant la séquence stérilité Ogura. Les plantes obtenues dans la descendance des méristèmes traités ou des plantules régénérées seront mâle-stériles cytoplasmiques si l'ADN a pu pénétrer dans les mitochondries et s'intégrer au génôme de ces organites. On évitera ainsi les problèmes posés par les séquences indésirables, qu'il s'agisse des chloroplastes du radis Ogura ou des séquences ainsi définies du génôme mitochondrial Ogura.

### EXEMPLE 6

Cet exemple illustre l'intérêt de la connaissance de la séquence "stérilité Ogura" dans la construction par génie génétique d'une stérilité mâle nucléaire, à hérédité mendélienne et non plus cytoplasmique.

A partir de la séquence d'ADN mitochondrial délimitée par les nucléotides 928 et 1569, on peut construire un gène chimérique qui sera après transformation génétique de cellules de Brassica ou d'un autre genre, transcrit dans le noyau des cellules des plantes transformées obtenues. Si le gène chimérique contient une préséquence qui permet l'importation dans la mitochondrie de son produit de traduction en protéine, ces transformants seront mâle-stériles, et ce caractère se comportera comme un caractère mendélien dominant.

### REFERENCES

Amann E, Ochs B, Abel K-J (1988) Gene 69:301-315
Bannerot H, Boulidard L, Cauderon Y, Tempé J (1974) Proc Eucarpia Meeting Cruciferae 25:52-54
Bannerot H, Boulidard L, Chupeau Y (1977) Eucarpia Cruciferae Newsl: 2-16 Chétrit P, Mathieu C, Vedel F, Pelletier G, Primard C (1985) Theor Appl Genet 69:361-366
Dellaporta SL, Wood J, Hicks JB (1983) Plant Mol Biol Rep r:19-21
Ducruet JM and Gasquez J (1978) Chemosphere 8:691-696
Frishauf AM, Lehrach H, Poutska A, Murray N (1983) J Mol Biol 170:827-842 Gough J and Murray N (1983) J Mol Biol 166:1-19
Hiesel R, Shobel W, Schuster W, Brennicke A (1987) EMBO J 6:29-34
Johnston SA, Anziano PQ, Shark K, Sanford JC, Butow RA (1988) Science 240:1538-1541
Logemann J, Schell J, Willmitzer L (1987) Analytical Biochem 163:16-20 Ogura H (1968 Mem Fac Agric Kagoshima Univ 6:39-78
Pelletier G, Primard C, Vedel F, Chétrit P, Rémy R, Rousselle P, Renard M (1983) Mol Gen Genet 191:244-250
Sambrook J, Fritsch EF, Maniatis T (1989) Molecular cloning, Cold Spring Harbor Laboratory, Cold Spring Harbor, New York
Stern DB and Newton KJ (1986) Methods Enzymol 118:488-496
Vedel F and Mathieu C (1982) Anal Biochem 127:1-8

## Revendications

1. Plante appartenant au genre Brassica, **caractérisée en ce qu'**elle comporte un génome mitochondrial ou nucléaire recombiné, ledit génome mitochondrial ou nucléaire possédant une séquence d'ADN stérilité Ogura qui consiste en
a) une séquence délimitée par les nucléotides numérotés 928 et 2273 sur la figure 1, ou
b) une séquence présentant au moins 50 % d'homologie avec ladite séquence mentionnée en a),
et qui confère une stérilité mâle cytoplasmique à ladite plante.

2. Plante selon la revendication 1 **caractérisée en ce que** ledit génome nucléaire ou mitochondrial recombiné comporte
a) une séquence d'ADN délimitée par les nucléotides numérotés 928 et 1569 de la figure 1,
b) ou une séquence ayant au moins 50% d'homologie avec ladite séquence mentionnée en a),
et qui confère une stérilité mâle cytoplasmique à ladite plante.

3. Plante selon l'une des revendications 1-2 **caractérisée en ce que** ledit génome nucléaire comporte en outre une préséquence permettant l'importation dans les mitochondries du produit de traduction de ladite séquence.

4. Plante selon l'une des revendications 1-3 **caractérisée en ce que** ledit génome nucléaire ou mitochondrial recombiné ne comporte pas tout ou une partie de l'un et/ou l'autre des deux fragments du génome Ogura :
- portant l'un des deux gènes de l'ARN de transfert formylméthionine servant à l'initiation de la traduction,
- portant le gène cox1, codant pour la sous unité n°1 de la cytochrome oxydase,
ou dans lequel lesdits fragments sont inactifs.

5. Plante selon l'une des revendications 2-4 **caractérisée en ce que** ledit génome nucléaire ou mitochondrial recombiné est dépourvu d'une partie de la séquence qui est portée par un fragment de 10.7 kb après digestion par BglI, et un fragment de 11 kb après digestion par NruI, révélés par hybridation avec une sonde coxl.

6. Plante selon l'une des revendications 2-5 **caractérisée en ce que** ledit génome nucléaire ou mitochondrial recombiné est dépourvu d'une partie de la séquence qui est portée après digestion par Nrul donne un fragment de 15 kb, après digestion par Sall donne un fragment de 5,1 kb, et après digestion par BglI donne un fragment de 18,5 kb, et qui est révélé par hybridation avec une sonde correspondant à la séquence comprise entre les bases 389 à 1199 de la séquence représentée sur la figure 1.

7. Plante selon l'une des revendications 2-6 **caractérisée en ce que** ledit génome nucléaire ou mitochondrial recombiné comporte une séquence qui après digestion par NcoI donne un fragment de 2,5 kb, après digestion par NruI donne un fragment de 6.8 kb, et après digestion par SalI donne un fragement de 4,4 kb.

8. Plante selon l'une des revendications 1-7 **caractérisée en ce qu'**elle appartient à l'une des espèces suivantes : B. napus, B. oleracea, B. campestris, B. nigra, B. juncea, B. hirta et B. carinata.

9. Plante selon l'une des revendications 1-8 **caractérisée en ce qu'**elle est obtenue par fusion de protoplastes.

10. Plante selon l'une des revendications 1-8 **caractérisée en ce qu'**elle est obtenue par reproduction sexuée.

11. Plante selon l'une des revendications 1-8 **caractérisée en ce que** son génome mitochondrial recombiné est obtenue par transfert de gène dans la mitochondrie.

12. Procédé de préparation de plantes hybrides qui consiste en les étapes suivantes :
a) Préparation d'une plante avec un génome mitochondrial ou nucléaire recombiné et présentant le caractère mâle stérile selon l'une des revendications 1-8,
b) Croisement de la plante obtenue en a) avec une plante de la même espèce, possédant éventuellement un gène restaurateur de fertilité Rf1.

13. Plante susceptible d'être obtenue par le procédé selon la revendication 12 et **caractérisée en ce qu'**elle comporte une séquence définie selon l'une des revendications 1 ou 2, a) ou b).

## Claims

1. A plant belonging to the *Brassica* genus, **characterized in that** it includes a recombinant nuclear or mitochondrial genome, said nuclear or mitochondrial genome having an Ogura sterility DNA sequence, which consists in
a) a sequence delimited by the nucleotides numbered 928 and 2273 in Fig. 1, or
b) a sequence having at least 50% homology with said sequence mentioned under a),
and which imparts cytoplasmic male sterility to said plant.

2. The plant according to claim 1, **characterized in that** said recombinant nuclear or mitochondrial genome includes
a) a DNA sequence delimited by the nucleotides numbered 928 and 1569 of Fig. 1,
b) or a sequence having at least 50% homology with said sequence mentioned under a),
and which imparts cytoplasmic male sterility to said plant.

3. The plant according to any of claims 1 to 2, **characterized in that** said nuclear genome further includes a presequence allowing the translation product of said sequence to be imported into mitochondria.

4. The plant according to any of claims 1 to 3, **characterized in that** said recombinant nuclear or mitochondrial genome does not include all or part of one and/or the other of both fragments of the Ogura genome:
- bearing one of the two genes for the formylmethionine transfer ARN being used for initiating the translation,
- bearing the cox1 gene, coding for the subunit No.1 of cytochrome oxidase,
or wherein said fragments are inactive.

5. The plant according to any of claims 2 to 4, **characterized in that** said recombinant nuclear or mitochondrial genome is lacking a portion of the sequence which is borne by a 10.7 kb fragment after digestion by BgII, and a 11 kb fragment after digestion by NruI, revealed by hybridization with a cox1 probe.

6. The plant according to any of claims 2 to 5, **characterized in that** said recombinant nuclear or mitochondrial genome is lacking a portion of the sequence which is borne after digestion by NruI gives a 15 kb fragment, after digestion by SalI gives a 5.1 kb fragment and after digestion by BglI gives a 18.5 kb fragment and which is revealed by hybridization with a probe corresponding to the sequence comprised between the bases 389 to 1199 of the sequence illustrated in Fig. 1.

7. The plant according to any of claims 2 to 6, **characterized in that** said recombinant nuclear or mitochondrial genome includes a sequence which after digestion by NcoI gives a 2.5 kb fragment, after digestion by NruI gives a 6.8 kb fragment, and after digestion by SalI gives a 4.4 kb fragment.

8. The plant according to any of claims 1 to 7, **characterized in that** it belongs to one of the following species: *B. napus, B. orleracea, B. campestris, B. nigra, B. juncea, B. hirta* and *B. carinata.*

9. The plant according to any of claims 1 to 8, **characterized in that** it is obtained by fusion of protoplasts.

10. The plant according to any of claims 1 to 8, **characterized in that** it is obtained by sexual reproduction.

11. The plant according to any of claims 1 to 8, **characterized in that** its recombinant mitochondrial genome is obtained by gene transfer in the mitochondrion.

12. A method for preparing hybrid plants which consists in the following steps:
a) preparing a plant with a recombinant mitochondrial or nuclear genome and having the sterile male character according to any of claims 1 to 8,
b) cross-breeding the plant obtained under a) with a plant of the same species, possibly having a fertility-restoring gene Rf1.

13. A plant capable of being obtained by the method according to claim 12 and **characterized in that** it includes a sequence defined according to any of claims 1 or 2; a) or b).

## Patentansprüche

1. Zu der Gattung Brassica gehörende Pflanze, **dadurch gekennzeichnet, dass** sie ein rekombiniertes mitochondriales oder nukleäres Genom umfasst, wobei das mitochondriale oder nukleäre Genom eine Ogura-Sterilitäts-DNA-Sequenz aufweist, welche aus
a) einer Sequenz, die durch die Nukleotide mit den Nummern 928 und 2273 in der Figur 1 begrenzt wird, oder
b) einer Sequenz, welche mindestens 50% Homologie mit der in a) erwähnten Sequenz aufweist,
besteht und welche der Pflanze eine zytoplasmatische männliche Sterilität verleiht.

2. Pflanze nach Anspruch 1, **dadurch gekennzeichnet, dass** das rekombinierte nukleäre oder mitochondriale Genom
a) eine DNA-Sequenz, welche durch die Nukleotide mit den Nummern 928 und 1569 der Figur 1 begrenzt wird,
b) oder eine Sequenz, welche mindestens 50% Homologie mit der in a) erwähnten Sequenz aufweist,
und welche der Pflanze eine zytoplasmatische männliche Sterilität verleiht, umfasst.

3. Pflanze nach einem der Ansprüche 1-2, **dadurch gekennzeichnet, dass** das nukleäre Genom außerdem eine Präsequenz, welche den Import des Translationsprodukts der genannten Sequenz in die Mitochondrien erlaubt, umfasst.

4. Pflanze nach einem der Ansprüche 1-3, **dadurch gekennzeichnet, dass** das rekombinierte nukleäre oder mitochondriale Genom nicht die Gesamtheit oder einen Teil des einen und/oder anderen der beiden Fragmente des Ogura-Genoms:
- welches eines der beiden Gene der Formylmethionin-Transfer-RNA, welche für die Initiation der Translation dient, trägt,
- welches das Gen cox1, welches die Untereinheit 1 der Cytochromoxidase kodiert, trägt, umfasst oder in welchem die genannten Fragmente inaktiv sind.

5. Pflanze nach einem der Ansprüche 2-4, **dadurch gekennzeichnet, dass** das rekombinierte nukleäre oder mitochondriale Genom frei ist von einem Teil der Sequenz, die sich auf einem Fragment von 10,7 kb nach Verdau durch BgII und einem Fragment von 11 kb nach Verdau durch Nrul, welche durch Hybridisierung mit einer cox1-Sonde nachgewiesen werden, befindet.

6. Pflanze nach einem der Ansprüche 2-5, **dadurch gekennzeichnet, dass** das rekombinierte nukleäre oder mitochondriale Genom frei ist von einem Teil der Sequenz, die sich befindet nach Verdau durch Nrul ein Fragment von 15 kb ergibt, nach Verdau durch Sall ein Fragment von 5,1 kb ergibt und nach Verdau durch BgII ein Fragment von 18,5 kb ergibt und die durch Hybridisierung mit einer Sonde, welche der Sequenz zwischen den Basen 389 bis 1199 eingeschlossen der in der Figur 1 dargestellten Sequenz entspricht, nachgewiesen wird.

7. Pflanze nach einem der Ansprüche 2-6, **dadurch gekennzeichnet, dass** das rekombinierte nukleäre oder mitochondriale Genom eine Sequenz umfasst, die nach Verdau durch NcoI ein Fragment von 2,5 kb ergibt, nach Verdau durch Nrul ein Fragment von 6,8 kb ergibt und nach Verdau durch Sall ein Fragment von 4,4 kb ergibt.

8. Pflanze nach einem der Ansprüche 1-7, **dadurch gekennzeichnet, dass** sie zu einer der folgenden Arten gehört: B. napus, B. oleracea, B. campestris, B. nigra, B. juncea, B. hirta und B. carinata.

9. Pflanze nach einem der Ansprüche 1-8, **dadurch gekennzeichnet, dass** sie durch Fusion von Protoplasten erhalten wird.

10. Pflanze nach einem der Ansprüche 1-8, **dadurch gekennzeichnet, dass** sie durch geschlechtliche Fortpflanzung erhalten wird.

11. Pflanze nach einem der Ansprüche 1-8, **dadurch gekennzeichnet, dass** ihr rekombiniertes mitochondriales Genom durch Gentransfer in das Mitochondrion erhalten wird.

12. Verfahren zur Herstellung von hybriden Pflanzen, das aus den folgenden Schritten besteht:
a) Herstellung einer Pflanze mit einem rekombinierten mitochondrialen oder nukleären Genom und welches den männlich sterilen Charakter gemäß einem der Ansprüche 1-8 aufweist,
b) Kreuzung der in a) erhaltenen Pflanze mit einer Pflanze der gleichen Art, welche gegebenenfalls ein Fertilitätswiederherstellungsgen Rf1 aufweist.

13. Pflanze, welche durch das Verfahren nach Anspruch 12 erhalten werden kann und **dadurch gekennzeichnet ist, dass** sie eine Sequenz, die gemäß einem der Ansprüche 1 oder 2 a) oder b) definiert ist, umfasst.
